(19) Europäisches Patentamt
European Patent Office
Office européen des brevets

(11) **EP 1 544 672 B1**

(12) **EUROPÄISCHE PATENTSCHRIFT**

(45) Veröffentlichungstag und Bekanntmachung des Hinweises auf die Patenterteilung:
**19.11.2008   Patentblatt 2008/47**

(51) Int Cl.:
*G03B 42/04* *(2006.01)*   *G01T 1/29* *(2006.01)*
*G06K 7/00* *(2006.01)*

(21) Anmeldenummer: **03104747.5**

(22) Anmeldetag: **17.12.2003**

(54) **Bildträger zur Speicherung von Röntgeninformation sowie System und Verfahren zur Bearbeitung eines solchen Bildträgers**

Radiation image storage medium as well as system for and method of processing such medium

Support d'image radiographique ainsi que système et méthode de traitement de ce support d'image

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**22.06.2005   Patentblatt 2005/25**

(73) Patentinhaber: **Agfa-Gevaert HealthCare GmbH
51373 Leverkusen (DE)**

(72) Erfinder:
• **Encke, Walter Dr.
82205, Gilching (DE)**
• **Haug, Werner
81825, München (DE)**

• **Hujer, Oskar
85305, Jetzendorf (DE)**

(74) Vertreter: **Linsmeier, Josef
Agfa-Gevaert HealthCare GmbH
Intellectual Property
Tegernseer Landstrasse 161
81539 München (DE)**

(56) Entgegenhaltungen:
**EP-A- 0 727 696        EP-A- 1 209 517
US-A- 4 498 005        US-A- 4 739 480
US-A- 5 418 355**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren zur Bearbeitung eines Bildträgers zur Aufzeichnung von Röntgenaufnahmen gemäß dem Oberbegriff des Anspruchs 1 sowie ein entsprechendes System zur Bearbeitung eines solchen Bildträgers gemäß dem Oberbegriff des Anspruchs 7.

Entsprechende Bildträger werden, insbesondere für medizinische Zwecke, im Bereich der Computer-Radiographie (CR) eingesetzt. Hierbei werden Röntgenaufnahmen in einer Speicherleuchtstoff-Schicht aufgezeichnet, indem die durch ein Objekt, beispielsweise einen Patienten, hindurchtretende Röntgenstrahlung als latentes Bild in der Speicherleuchtstoff-Schicht gespeichert wird. Zum Auslesen der gespeicherten Röntgeninformation wird die Speicherleuchtstoff-Schicht mit Stimulationslicht bestrahlt, wodurch diese zur Aussendung von Emissionslicht angeregt wird, welches von einem optischen Detektor erfasst und in elektrische Signale umgewandelt wird. Die elektrischen Signale können nach Bedarf weiterverarbeitet und auf einem Monitor dargestellt oder an einem entsprechenden Ausgabegerät, wie z. B. einem Drucker, ausgegeben werden. Nach einem Löschvorgang, bei dem etwaige verbliebene Röntgeninformation vollständig aus der Speicherleuchtstoff-Schicht eliminiert wird, steht die Speicherleuchtstoff-Schicht für weitere Röntgenaufnahmen zur Verfügung.

Neben einer Speicherleuchtstoff-Schicht zur Speicherung von Röntgeninformation weisen Bildträger nach dem Stand der Technik beispielsweise einen integrierten elektronischen Schaltkreis mit einem Speicher auf, in welchem dem Bildträger und/oder einer Röntgenaufnahme zuzuordnende Informationen gespeichert werden können. Diese Informationen werden bei einer anschließenden Bearbeitung des Bildträgers in einer Bearbeitungsvorrichtung, beispielsweise einer Auslesevorrichtung zum Auslesen der Speicherleuchtstoff-Schicht, herangezogen.

Beim Einsatz solcher Bildträger, die aufgrund ihrer Wiederbeschreibbarkeit im Allgemeinen eine große Zahl von Radiographie-Zyklen durchlaufen, kann es immer wieder vorkommen, dass der integrierte Schaltkreis ausfällt und die darin gespeicherten Daten nicht mehr ausgelesen werden können und folglich verloren gehen. Eine Bearbeitung dieses Bildträgers, für welche diese Daten erforderlich sind, kann dann nicht mehr oder nicht mehr ausreichend zuverlässig durchgeführt werden.

Bei dem aus US 5,418,355 bekannten System befinden sich auf der Kassette sowohl ein Barcode als auch ein nicht-flüchtiger Schreib-/Lese-Speicher. Die im Barcode enthaltene Information wird mittels eines geeigneten Schreibgerätes zum Speicher übertragen. Aus EP 1 209 517 A2 ist bekannt, dass der Inhalt eines auf der Kassette befindlichen Barcode-Labels mit dem Inhalt eines per Radiofrequenzstrahlung auslesbaren Elements übereinstimmen sollte. Die Verwendung solcher Elemente, sog. RF-Tags, bei Röntgenkassetten ist aus EP 0 727 696 A1 bekannt.

Bei den bekannten Verfahren und Systemen kann im Falle eines defekten Speichers bzw. RF-Tags ein Datenverlust auftreten.

Es ist Aufgabe der vorliegenden Erfindung, ein Verfahren und entsprechendes System zur Bearbeitung des Bildträgers anzugeben, bei welchem die Gefahr eines Datenverlustes vermindert wird.

Diese Aufgabe wird durch das Verfahren bzw. das System gemäß den unabhängigen Ansprüchen gelöst.

Die Erfindung zeichnet sich insbesondere dadurch aus, dass der Bildträger eine Markierung aufweist, welche zumindest einen Teil der im elektronischen Speicher gespeicherten Daten repräsentiert. Die Markierung ist beispielsweise als Etikett, welches die repräsentierten Daten trägt und am Bildträger angebracht ist, oder als entsprechender Aufdruck auf dem Bildträger ausgebildet.

Durch die Erfindung wird auf einfache Weise sichergestellt, dass der im elektronischen Speicher gespeicherte und in der Markierung enthaltene Teil der Daten auch im Falle eines Defekts des Speichers nicht verloren geht. In einem solchen Fall kann der defekte elektronische Speicher einfach gegen einen neuen ausgetauscht werden, in welchen dann die der Markierung entnommenen Daten geschrieben werden können. Diese Daten können dadurch wieder aus dem - nunmehr neuen - integrierten Schaltkreis ausgelesen werden und stehen damit bei weiteren Bearbeitungen des Bildträgers weiter zur Verfügung.

Der elektronische Speicher ist vorzugsweise als integrierter elektronischer Schaltkreis mit einem nicht-flüchtigen Speicher ausgebildet, beispielsweise als ROM, PROM, EPROM oder EEPROM.

In einer ersten bevorzugten Ausgestaltung gibt die Markierung die Daten in Klarschrift wieder, welche vorzugsweise mit dem bloßen Auge lesbar ist. Die Markierung umfasst dabei insbesondere eine alphanumerische Zeichenfolge. Hierdurch kann sie von einer Bedienperson einfach wahrgenommen, gelesen und entziffert werden, ohne dass zusätzliche Einrichtungen zum Lesen bzw. Dekodieren erforderlich wären.

Alternativ oder zusätzlich kann die Markierung die Daten auch in maschinenlesbarer Form, vorzugsweise als Barcode, wiedergeben. Hierdurch wird ein schnelles und wenig fehleranfälliges Einlesen der durch die Markierung repräsentierten Daten gewährleistet.

In einer Ausgestaltung der Erfindung ist vorgesehen, dass die Daten im elektronischen Speicher in unterschiedlichen Datengruppen gespeichert sind und die Markierung die im elektronischen Speicher gespeicherten Daten von zumindest einer dieser Datengruppen repräsentiert. Hierdurch wird zum einen eine übersichtliche Aufteilung des Speicherinhalts in Daten, die zusätzlich durch die Markierung repräsentiert werden, und Daten, die nicht in der Markierung enthalten sind, erreicht. Darüber hinaus wird ein einfacherer Schreib- und/oder Lesezugriff auf die Daten einer einzelnen Datengruppen ermöglicht.

Bei den unterschiedlichen Datengruppen handelt es sich um eine oder mehrere der folgenden Datengruppen:

- eine Bildträger-Datengruppe mit für den Bildträger spezifischen Daten, z.B. zur Identifizierung sowie zu Eigenschaften des Bildträgers;
- eine Zustands-Datengruppe mit Daten zu einem Bearbeitungszustand des Bildträgers;
- eine Steuerungs-Datengruppe mit Daten zur Steuerung des Auslesens und/oder Löschens der im Bildträger gespeicherten Röntgeninformation in einer Auslesevorrichtung;
- eine Verarbeitungs-Datengruppe mit Daten zur Steuerung einer Weiterverarbeitung und/oder Wiedergabe der ausgelesenen Röntgeninformation des Bildträgers in einer Wiedergabevorrichtung;
- eine Kalibrierungs-Datengruppe mit Daten zur Kalibrierung des Bildträgers, welche für eine Vorverarbeitung der ausgelesenen Röntgeninformation des Bildträgers, insbesondere in einer Auslesevorrichtung, herangezogen werden;
- eine Patienten-Datengruppe mit Daten zu einem Patienten, dessen Röntgeninformation im Bildträger gespeichert ist, z.B. Daten zur Identifizierung des Patienten.

Vorzugsweise repräsentiert die Markierung die im elektronischen Speicher gespeicherten Daten der Bildträger-Datengruppe, welche für den Bildträger spezifische Daten umfasst. Die Daten dieser Datengruppe werden in der Regel bereits bei der Herstellung des Bildträgers in den elektronischen Speicher geschrieben und enthalten unter anderem Informationen zur eindeutigen Identifizierung des Bildträgers. Ein Verlust dieser Daten würde dazu führen, dass der Bildträger für eine weitere Verwendung unbrauchbar wird, da die Daten wegen der unmöglichen Identifizierung des Bildträgers dann auch nicht mehr beim Hersteller beschafft werden können. Die durch die Markierung erreichbare Schadensminimierung im Hinblick auf Datenverluste ist bei dieser Ausgestaltungsform der Erfindung dadurch besonders deutlich.

Die Bildträger-Datengruppe umfasst insbesondere eines oder mehrere der folgenden Daten:

- Initialisierungsdatum, welches dem Datum entspricht, an dem die Daten der Bildträger-Datengruppe in den Speicher des Bildträgers geschrieben wurden;
- Bildträger-Seriennummer, welche eine eindeutige Identifizierung des Bildträgers erlaubt und sich vorzugsweise aus einem Code, der die Produktcharge des jeweiligen Bildträgers identifiziert, und einer laufenden Nummer zusammengesetzt;
- Größe des Bildträgers;
- Größe einer auszulesenden Fläche auf dem Bildträger;

- Typ des Bildträgers;
- Empfindlichkeit des Bildträgers;
- Löscheigenschaft des Bildträgers, insbesondere als Maß für die Dauer und/oder Intensität von von einer Löscheinrichtung abgegebenem Licht.

In einer weiteren vorteilhaften Ausgestaltung umfasst der Bildträger eine Bildplatte zur Speicherung der Röntgeninformation, wobei die Markierung an der Bildplatte angebracht ist. Dadurch wird eine zuverlässige Zuordnung der Markierung zur Bildplatte gewährleistet.

In einer vorteilhaften Ausführung dieser Variante ist vorgesehen, dass die Bildplatte eine Trägerschicht mit einer auf der Vorderseite der Trägerschicht befindlichen Speicherleuchtstoff Schicht umfasst und die Markierung in einem Randbereich der Trägerschicht, insbesondere außerhalb der Speicherleuchtstoff-Schicht, und/oder an der der Vorderseite gegenüberliegenden Rückseite der Trägerschicht angebracht ist. Hierdurch wird eine Beeinträchtigung der Speichereigenschaften der Speicherleuchtstoff-Schicht durch die Markierung vermieden.

In der Regel umfasst der Bildträger neben der Bildplatte eine Kassette, welche die Bildplatte unter Abschirmung von Umgebungslicht aufnehmen kann.

In einer alternativen Ausgestaltung der Erfindung ist die Markierung an der Kassette angebracht. Sie kann dann einfach von außen, d.h. von der Außenseite der Kassette her, gelesen werden, ohne dass die Kassette geöffnet und die Bildplatte entnommen werden müsste.

Bei dem erfindungsgemäßen Verfahren zur Bearbeitung eines entsprechenden Bildträgers ist vorgesehen, dass die von einer Markierung auf dem Bildträger repräsentierten Daten in den elektronischen Speicher geschrieben werden.

Bei einem Defekt wird hierbei ein erster elektronischer Speicher durch einen zweiten elektronischen Speicher ersetzt und die in der Markierung enthaltenen Daten, die einen Teil der im ersten elektronischen Speicher gespeicherten Daten repräsentieren, in den zweiten elektronischen Speicher geschrieben.

Das entsprechende erfindungsgemäße System zur Bearbeitung eines Bildträgers zur Speicherung von Röntgeninformation besteht aus einem Bildträger, welcher einen elektronischen Speicher zur Speicherung von Daten umfasst, und einer ID-Station, an welcher Daten eingegeben und in den elektronischen Speicher geschrieben werden können, wobei die ID-Station auch die von einer Markierung auf dem Bildträger repräsentierten Daten in den elektronischen Speicher schreiben kann.

In Fällen, in denen der auf dem Bildträger befindliche elektronische Speicher defekt ist und gegen einen neuen elektronischen Speicher ausgetauscht wird, können mit dem erfindungsgemäßen System die in der Markierung enthaltenen Daten, die einen Teil der im defekten elektronischen Speicher enthaltenen Daten repräsentieren, in den neuen elektronischen Speicher geschrieben werden. Die ID-Station umfasst hierzu eine geeignete Eingabeeinrichtung, wie z.B. eine Tastatur und/oder ein Lesegerät, über welche bzw. welches die durch die Markierung repräsentierten Daten eingegeben bzw. eingelesen werden können, sowie eine Schreibeinrichtung, welche die eingegebenen bzw. eingelesenen Daten in den elektronischen Speicher schreiben kann.

Weitere Merkmale und Vorteile der Erfindung ergeben sich aus der folgenden Beschreibung bevorzugter Ausführungsformen und Anwendungsbeispiele, wobei Bezug auf die beigefügten Figuren genommen wird.

Es zeigen:

Fig. 1   ein System zur Bearbeitung eines Bildträgers für Röntgeninformation;

Fig. 2   ein erstes Beispiel einer Struktur der im integrierten Schaltkreis des Bildträgers gespeicherten Daten;

Fig. 3   eine schematische Darstellung einer ersten Variante des Datenflusses zwischen einzelnen Komponenten des in Fig. 1 dargestellten Systems;

Fig. 4   eine schematische Darstellung einer zweiten Variante des Datenflusses zwischen einzelnen Komponenten des in Fig. 1 dargestellten Systems; und

Fig. 5   ein zweites Beispiel einer Struktur der im integrierten Schaltkreis des Bildträgers gespeicherten Daten.

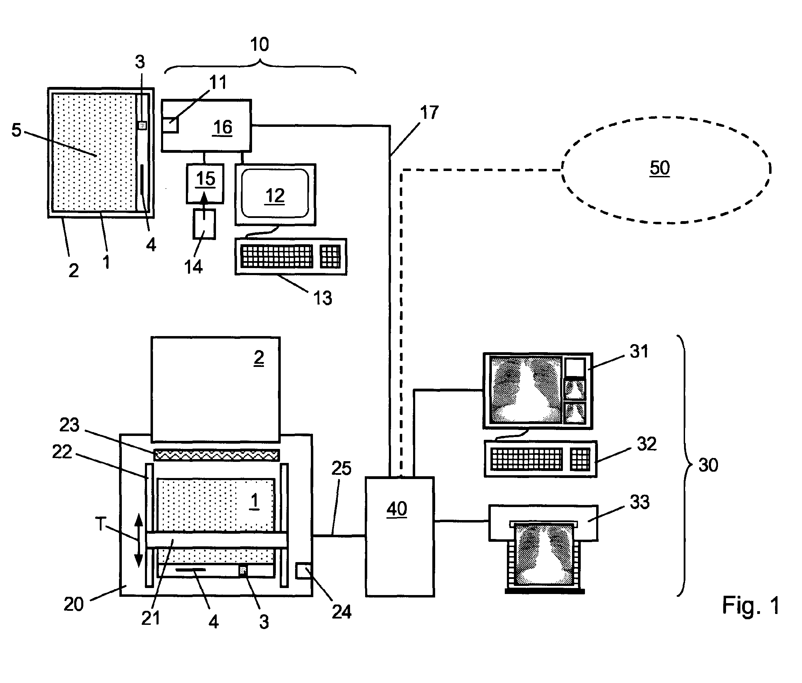

Fig. 1 zeigt ein System zur Bearbeitung eines Bildträgers für Röntgeninformation. Das System umfasst eine Identifikationsstation 10, welche im Folgenden auch als ID-Station bezeichnet wird, eine Auslesevorrichtung 20, eine Wiedergabevorrichtung 30 sowie einen Zentralspeicher 40.

Der Bildträger für Röntgeninformation besteht aus einer Kassette 2 mit einer darin befindlichen Bildplatte 1. Die Bildplatte 1 umfasst eine Trägerschicht mit einer darauf aufgebrachten Speicherleuchtstoff-Schicht 5. Die Speicherleuchtstoff-Schicht 5 weist vorzugsweise einen Speicheneuchtstoff auf der Basis von BaFBr:Eu oder CsBr:Eu auf.

Die Bildplatte 1 ist mit einem integrierten Schaltkreis 3 versehen, der einen Schreib-Lese-Speicher umfasst, in welchen Daten geschrieben und aus welchem Daten gelesen werden können. Um die Speicherfunktion der SpeicherleuchtstoffSchicht 5 nicht zu beeinträchtigen, wird der integrierte Schaltkreis 3 vorzugsweise an der Rückseite oder - wie im dargestellten Beispiel - im Randbereich der Bildplatte 1 angeordnet.

Alternativ oder zusätzlich kann der integrierte Schaltkreis 3 an der Kassette 2 angebracht sein. Die folgenden Ausführungen, die sich auf einen an der Bildplatte 1 befindlichen integrierten Schaltkreis 3 beziehen, gelten auch für diese Alternative entsprechend.

Nach einer Röntgenaufnahme wird die in der Kassette 2 befindliche Bildplatte 1, in deren Speicheneuchtstoff-Schicht 5 Röntgeninformation gespeichert ist, zur ID-Station 10 gebracht, um Daten aus dem und/ oder in den integrierten Schaltkreis 3 zu lesen bzw. zu schreiben. Die Datenübertragung zwischen dem integrierten Schaltkreis 3 und der ID-Station 10 erfolgt vorzugsweise kontaktlos. Hierdurch kann eine exakte Positionierung der Bildplatte 1 und des Schaltkreises 3 relativ zur ID-Station 10, wie sie bei einer kontaktbehafteten Datenübertragung erforderlich wäre, entfallen. Die kontaktlose Datenübertragung erfolgt vorzugsweise mittels Radiofrequenz-Wellen (RF-Wellen). Zu diesem Zweck weist die ID-Station 10 eine entsprechende erste Schreib-Lese-Einrichtung 11 mit einem RF-Sender und einem RF-Empfänger auf.

Der integrierte Schaltkreis 3 ist vorzugsweise in Form eines sogenannten RF-Etiketts, welches auch als RF-Tag bezeichnet wird, an der Bildplatte 1 angebracht, beispielsweise aufgeklebt. Ein solches RF-Tag umfasst neben dem integrierten Schaltkreis 3 eine als Transponderspule ausgebildete Antenne. Statt als RF-Tag kann der integrierte Schaltkreis 3 auch in Form einer Chipkarte, bei welcher ein RF-Tag in einen kartenförmigen Plastikkörper eingebracht ist, an der Bildplatte 1 angebracht sein. Die Chipkarte wird vorzugsweise lösbar an der Bildplatte 1 befestigt, z.B. mittels einer einfachen Steckverbindung im Randbereich der Bildplatte 1. Zur zusätzlichen Sicherung der Chipkarte kann ein Arretiermechanismus vorgesehen sein, z.B. ein Schnappmechanismus, bei welchem die Chipkarte durch Zuschnappen arretiert wird. Diese Befestigung der Chipkarte an der Bildplatte 1 ist einerseits einfach und sicher und vereinfacht andererseits den - beispielsweise im Falle eines Defekts des integrierten Schaltkreises 3 erforderlichen - Austausch der Chipkarte gegen eine andere.

Die Bildplatte 1 ist mit einer mit dem bloßen Auge lesbaren Markierung 4 versehen, welche zumindest einen Teil der im Speicher des integrierten Schaltkreises 3 gespeicherten Daten, insbesondere für die Bildplatte 1 spezifische Daten, repräsentiert und vorzugsweise als alphanumerische Zeichenfolge ausgebildet ist. Hierdurch sind bei einem Defekt des integrierten Schaltkreises 3, bei dem die darin gespeicherten Daten im Allgemeinen nicht mehr ausgelesen werden können, die in der Markierung 4 enthaltenen Daten weiterhin zugänglich. Der defekte integrierte Schaltkreis 3 kann in diesem Fall gegen einen neuen ausgetauscht werden, in welchen dann die in der Markierung 4 enthaltenen Daten geschrieben werden. Hierzu werden die Daten der Markierung 4 von einem Bediener gelesen, an der ID-Station 10 eingegeben und schließlich in den neuen integrierten Schaltkreis geschrieben. Mit Hilfe der Markierung 4 wird damit auf einfache Weise sichergestellt, dass der im integrierten Schaltkreis 3 gespeicherte und in der Markierung 3 enthaltene Teil der Daten auch bei einem Defekt des Schaltkreises 3 nicht verlorengeht.

Alternativ oder zusätzlich kann eine derart ausgestaltete Markierung 4 auch an der Kassette 2 angebracht sein. Das Vorgehen bei einem Defekt des integrierten Schaltkreises erfolgt in analoger Weise.

Die ID-Station 10 umfasst eine oder mehrere Eingabeeinrichtungen zur Eingabe von Daten, welche beispielsweise für einen zu untersuchenden Patienten, das Auslesen der Bildplatte 1 oder die Weiterverarbeitung von aus der Bildplatte 1 ausgelesenen Bilddaten spezifisch bzw. erforderlich sind. Im dargestellten Beispiel umfassen die Eingabeeinrichtungen eine Tastatur 13 mit Anzeigeeinheit 12, wie z. B. einen Monitor, sowie einen Kartenleser 15 für eine Karte 14, auf welcher sich einzugebende Daten befinden, Insbesondere handelt es sich bei der Karte 14 um eine Chipkarte, beispielsweise eine Krankenversicherungskarte, auf welcher patientenspezifische Daten, wie z.B. Name, Adresse, Geburtsdatum und Versicherungsnummer eines Patienten, gespeichert sind. Der Kartenleser 15 liest diese Daten aus der Karte 14 aus und übergibt sie an einen Zwischenspeicher 16 der ID-Station 10. Auch die über die Tastatur 13 eingegebenen Daten werden an den Zwischenspeicher 16 übergeben. Bei der Anzeigeeinheit 12 kann es sich vorzugsweise auch um einen sog. Touchscreen handeln, bei welchem angezeigte Funktionen und/oder Daten durch Berührung der entsprechenden Bereiche der Anzeige ausgewählt werden können.

Vom Zwischenspeicher 16 aus werden die eingegebenen Daten an die erste Schreib-Lese-Einrichtung 11 übergeben und in den Speicher des integrierten Schaltkreises 3 auf der Bildplatte 1 geschrieben.

Anstelle der eingegebenen Daten selbst kann ein diesen Daten zugeordneter Referenzcode in den Speicher des integrierten Schaltkreises 3 geschrieben werden, während die eingegebenen Daten zusammen mit dem ihnen zugeordneten Referenzcode im Zwischenspeicher 16 und/oder im Zentralspeicher 40 gespeichert werden. Auf dieses Verfahren wird weiter unten noch näher eingegangen.

Anschließend wird die Kassette 2 mit der darin befindlichen Bildplatte 1 zur Auslesevorrichtung 20 gebracht, wo die Kassette 2 automatisch geöffnet, die Bildplatte 1 entnommen und in das Innere der Auslesevorrichtung 20 eingezogen wird. In der hier dargestellten Position ist die Bildplatte 1 bereits vollständig aus der Kassette herausgezogen und im Innern der Auslesevorrichtung 20 arretiert. In dieser Position wird sie von einem Scanner 21, welcher mit einem geeigneten Transportmechanismus 22 in Transportrichtung T über die Bildplatte 1 bewegt wird, ausgelesen.

Der Scanner 21 ist vorzugsweise als sog. Zeilenscanner ausgebildet, welcher eine zeilenförmige Stimulationslichtquelle, vorzugsweise mit in einer Reihe angeordneten Laserdioden, und einen zeilenförmigen Detektor, vorzugsweise ein lineares CCD-Array, aufweist. Während der Bewegung des Scanners 21 über die Bildplatte 1 wird diese mit dem Licht der Stimulationslichtquelle zeilenweise bestrahlt, wobei in der Speicherleuchtstoff-Schicht 5 Emissionslicht angeregt wird, dessen Intensität der in der Speicherleuchtstoff-Schicht 5 gespeicherten Röntgeninformation entspricht. Das Emissionslicht wird mit dem zeilenförmigen Detektor erfasst und in entsprechende Bildsignale umgewandelt. Durch die kontinuierliche Bewegung des Scanners 21 in Transportrichtung T über die Bildplatte 1 wird die Speicherleuchtstoff-Schicht 5 sukzessive zeilenweise ausgelesen, wobei ein zweidimensionales Abbild der gespeicherten Röntgeninformation erhalten wird.

Alternativ kann der Scanner 21 auch als sog. Flying Spot-Scanner ausgebildet sein, bei welchem ein einzelner Laserstrahl durch einen rotierenden Polygonspiegel auf die Speicherleuchtstoff Schicht 5 gelenkt wird, wodurch diese entlang einzelner Zeilen punktweise abgetastet wird.

Nachdem die Bildplatte 1 vollständig ausgelesen worden ist, wird diese wieder automatisch zurück in die Kassette 2 befördert. Dabei passiert sie eine Löscheinrichtung 23, an welcher etwaige restliche Röntgeninformationen in der Bildplatte 1 durch Bestrahlung der Speicherleuchtstoff-Schicht 5 mit Löschstrahlung gelöscht werden. Die Löscheinrichtung 23 umfasst hierzu eine Strahlungsquelle mit einem - verglichen mit der Stimulationstichtquelle - breitbandigerem Spektrum und einen geeigneten Reflektor zur Reflexion von Löschstrahlung auf die Speicherleuchtstoff-Schicht 5 der Bildplatte 1.

Die Auslesevorrichtung 20 umfasst eine zweite Schreib-Lese-Einrichtung 24, mit welcher Daten aus dem bzw. in den integrierten Schaltkreis 3 gelesen bzw. geschrieben werden können. Wie die erste Schreib-Lese-Einrichtung 11 der ID-Station 10 ist auch die zweite Schreib-Lese-Einrichtung 24 vorzugsweise zur kontaktlosen Datenübertragung, insbesondere mittels RF-Wellen, ausgebildet. Die mit der zweiten Schreib-Lese-Einrichtung 24 aus dem Speicher des integrierten Schaltkreises 3 ausgelesenen Daten werden insbesondere zur Steuerung des Auslesens der Bildplatte 1 mit dem Scanner 21 und/oder zur Steuerung des Löschens der Bildplatte 1 mit der Löscheinrichtung 23 herangezogen. Darüber hinaus können mit der zweiten Schreib-Lese-Einrichtung 24 Daten in den integrierten Schaltkreis 3 geschrieben werden, um vorzugsweise Daten zum Bearbeitungsstatus der Bildplatte 1 - beispielsweise, ob die Bildplatte 1 bereits ausgelesen bzw. gelöscht worden ist - zu aktualisieren.

Beim zeilenweisen Auslesen der Bildplatte 1 mit dem Scanner 21 werden Bilddaten erzeugt, welche die in der Bildplatte 1 gespeicherte Röntgeninformation repräsentieren. Diese Bilddaten werden über einen ersten Datenbus 25, insbesondere einen seriellen Datenbus, wie z.B. einen sog. FireWire, an den Zentralspeicher 40 übertragen und dort gespeichert.

Die im Zentralspeicher 40 gespeicherten Bilddaten können dann in der Wiedergabevorrichtung 30 weiterverarbeitet und/oder wiedergegeben werden. Zu diesem Zweck umfasst die Wiedergabevorrichtung 30 einen mittels einer Tastatur 32 ansteuerbaren Monitor 31. Alternativ oder zusätzlich kann auch ein Hardcopygerät 33, beispielsweise ein Laserdrucker, zur Ausgabe der Bilddaten vorgesehen sein.

Der Zentralspeicher 40 kann optional an einem Netzwerk 50, insbesondere an einem lokalen Netzwerk (LAN), angeschlossen sein. Hierdurch wird ermöglicht, dass von anderen Systemen aus, insbesondere von anderen ID-Stationen und/oder Wiedergabevorrichtungen aus, auf die im Zentralspeicher 40 abgespeicherten Daten bzw. Bilddaten zugegriffen werden kann.

Der Zentralspeicher 40 kann als separate zentrale Einheit ausgebildet sein, welche z.B. in einem zentralen File-Server integriert sein kann. Alternativ kann der Zentralspeicher 40 aber auch integraler Bestandteil der ID-Station 10, der Auslesevorrichtung 20 oder der Wiedergabevorrichtung 30 sein.

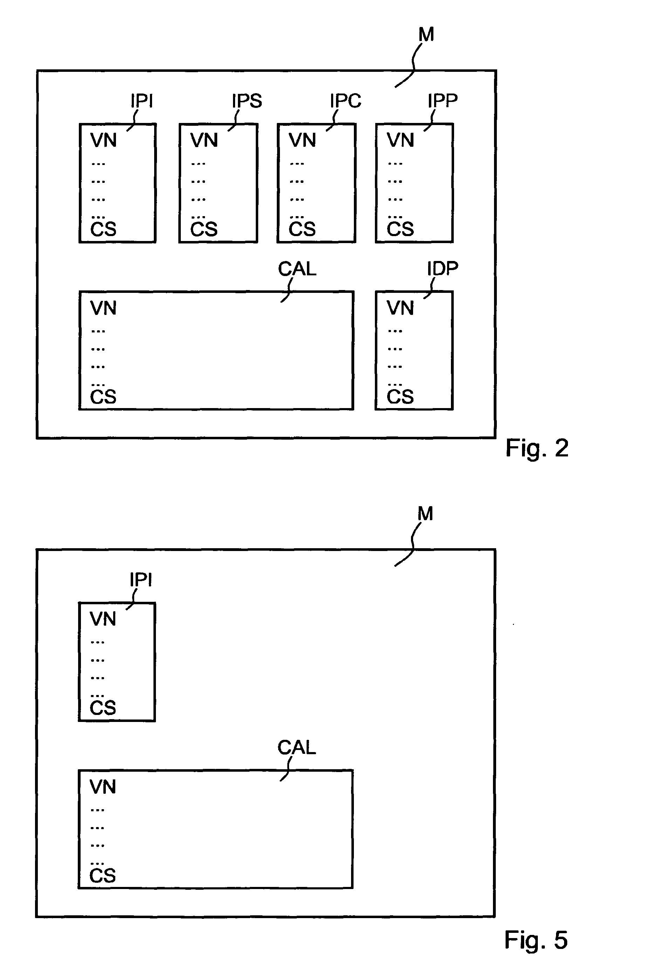

Fig. 2 zeigt ein erstes Beispiel einer Struktur der im Speicher M des integrierten Schaltkreises 3 des Bildträgers gespeicherten Daten. Anhand dieser Struktur wird nachfolgend die Datenübertragung innerhalb des in Fig. 1 beschriebenen Systems näher erläutert. Es ist zu beachten, dass die hier gewählte Darstellung die Struktur der Daten nur stark schematisiert wiedergibt und nicht auf eine bestimmte räumliche Anordnung oder eine bestimmte Reihenfolge der Daten im Speicher M des integrierten Schaltkreises 3 beschränkt ist.

Die im Speicher M gespeicherten Daten sind in unterschiedlichen Datengruppen IPI, IPS, IPC, IPP, CAL und IDP zusammengefasst und dort jeweils als ASCII-String abgelegt. Jeder Datengruppe ist eine Versionsnummer VN sowie eine Prüfsumme CS zugeordnet.

Die am Beginn jeder Datengruppe stehende Versionsnummer VN gibt an, in welcher Datenstruktur die Daten einer Datengruppe abgelegt sind. Hierdurch wird die Möglichkeit eröffnet, unterschiedliche Datenstrukturen ein und derselben Datengruppe vorzusehen. Dies ist immer dann erforderlich, wenn andere oder zusätzliche Daten im Speicher M abgelegt werden sollen, beispielsweise bei einem neuen Bildplattentyp oder einer neuen Art der Weiterverarbeitung der Bilddaten. Anhand der Versionsnummer VN einer Datengruppe können die einzelnen Komponenten des Systems, z.B. die ID-Station 10, die Auslesevorrichtung 20 bzw. die Wiedergabevorrichtung 30, erkennen, welche Daten in der jeweiligen Datengruppe enthalten sind und in welcher Datenlänge, Reihenfolge usw. diese Daten vorliegen.

Die am Ende jeder Datengruppe stehende Prüfsumme CS wird aus den einzelnen Daten dieser Datengruppe abgeleitet. Anhand der jeweiligen Prüfsumme CS kann festgestellt werden, ob die Daten dieser Datengruppe fehlerfrei gespeichert bzw. gelesen worden sind. Als Prüfsumme CS dient vorzugsweise der Rest der Division der Summe Σ der Zahlenwerte aller in dieser Datengruppe enthaltenen Bytes durch 256, also

$$CS = \Sigma \; Modulo \; 256.$$

Im Folgenden wird die Struktur und die Funktion der einzelnen Datengruppen IPI, IPS, IPC, IPP, CAL und IDP näher erläutert.

Eine Bildträger-Datengruppe IPI enthält spezifische Daten der Bildplatte 1 und/oder der Kassette 2. Diese Daten werden bei der Herstellung der Bildplatte 1 bzw. der Kassette 2 im Speicher M gespeichert und bleiben bei der Bearbeitung der Bildplatte 1 bzw. der Kassette 2 an der ID-Station 10 und/oder in der Auslesevorrichtung 20 unverändert, d.h. an der ID-Station 10 bzw. in der Auslesevorrichtung 20 werden die in der Bildträger-Datengruppe IPI gespeicherten Daten ausschließlich gelesen. Neben der Versionsnummer VN und der Prüfsumme CS umfasst die Bildträger-Datengruppe IPI folgende Daten:

- Initialisierungsdatum: entspricht dem Datum, an welchem die Daten der Bildträger-Datengruppe IPI in den Speicher M der Bildplatte 1 geschrieben wurden;
- Bildplatten-Seriennummer: dient zur eindeutigen Identifizierung der Bildplatte 1 und ist vorzugsweise aus einem die jeweilige Produktcharge der Bildplatte 1 identifizierenden Code und einer laufenden Nummer zusammengesetzt;
- Bildplattengröße;
- Größe einer auszulesenden Fläche auf der Bildplatte 1;
- Bildplattentyp: z.B. Bildplatte auf der Basis von pulverförmigen oder nadelförmigen Speicherleuchtstoffen, wie z.B. sogenannte Powder-IP bzw. Needle-IP;
- Bildplatten-Empfindlichkeit;
- Bildplatten-Löscheigenschaft: z.B. als Maß für die Dauer und/oder Intensität des von einer Löscheinrichtung 23 abgegebenen Lichts.

Vorzugsweise wird der Inhalt der Bildträger-Datengruppe IPI zusätzlich als visuell lesbare Markierung 4 (siehe Fig. 1) auf die Bildplatte 1 und/oder die Kassette 2 aufgebracht. Dies hat den Vorteil, dass die für die Bildplatte 1 bzw. Kassette 2 spezifischen und für deren Bearbeitung erforderlichen Daten der Bildträger-Datengruppe IPI auch dann nicht verlorengehen können, wenn der integrierte Schaltkreis 3 defekt ist und nicht mehr ausgelesen werden kann. In einem solchen Fall wird der defekte integrierte Schaltkreis 3 einfach gegen einen neuen integrierten Schaltkreis ausgetauscht, welcher dann mit den der Markierung 4 entnommenen Daten beschrieben wird. Eine solche Markierung 4 kann beispielsweise wie folgt lauten:

301 - 6KBQMF0001 - 20030702 -1 - 1 - 0 - 1000 - 1000 - 123

Die ersten drei (301) sowie die letzten drei (123) Zeichen beinhalten die Versionsnummer VN bzw. die Prüfsumme CS. Nach der Versionsnummer VN folgen die Bildplatten-Seriennummer (6KBQMF0001), das Initialisierungsdatum (20030702), Werte für die Bildplattengröße (1), die Größe (1) der auszulesenden Fläche der Bildplatte sowie den Bildplattentyp (0), die Bildplatten-Empfindlichkeit (1000) und schließlich die Bildplatten-Löscheigenschaft (1000).

Die der Markierung 4 entnommenen Daten können an der ID-Station 10 auf einfache Weise in den Speicher des neuen integrierten Schaltkreises geschrieben werden, ohne dass eine neue Initialisierung beim Hersteller der Bildplatte 1 bzw. Kassette 2 erforderlich wäre. Im einfachsten Fall werden die Daten der Markierung 4 von einem Bediener gelesen, über die Tastatur 13 der ID-Station 10 eingegeben und anschließend in den Speicher des neuen integrierten Schaltkreises geschrieben.

Die oben beschriebene visuell, d.h. mit dem menschlichen Auge, lesbare Markierung 4 stellt eine besonders einfache Möglichkeit zur zusätzlichen Sicherung von im integrierten Schaltkreis 3 gespeicherten Daten dar. Selbstverständlich kann anstelle einer visuell lesbaren Markierung alternativ oder zusätzlich eine maschinell lesbare Markierung (nicht dargestellt) vorgesehen sein, beispielsweise in Form eines Barcodes oder Magnetstreifens. Zur Eingabe der in der maschinell lesbaren Markierung enthaltenen Daten ist dann ein entsprechendes Barcode- bzw. Magnetstreifenlesegerät erforderlich, das die maschinell eingelesenen Daten an die ID-Station 10 weiterleitet, wo diese dann in den neuen integrierten Schaltkreis geschrieben werden können.

Die Daten einer Zustands-Datengruppe IPS betreffen den jeweiligen Bearbeitungszustand der Bildplatte 1 bzw. der Kassette 2 und werden sowohl in der ID-Station 10 als auch in der Auslesevorrichtung 20 geändert. Neben der Versionsnummer VN und der Prüfsumme CS umfasst diese Datengruppe folgende Daten:

- Bildplattenstatus: z.B. ob die Bildplatte 1 bereits initialisiert ist (d.h. Daten der Bildträger-Datengruppe IPI im Speicher M gespeichert sind) und/oder ein Röntgenbild bereits aufgenommen, gelöscht bzw. noch zu löschen ist;
- Bildplattenzyklen: Gesamtzahl von Röntgenaufnahmen mit dieser Bildplatte 1. Diese Gesamtzahl wird nach jedem Auslesevorgang oder Löschvorgang in der Auslesevorrichtung 20 um den Wert 1 erhöht;
- Auslesen unter Biegung: Anzahl der Bildplattenzyklen, in welchen die Bildplatte 1 während des Auslesevorgangs gekrümmt wird. Diese Zahl ist ein Maß für den Abnutzungs- und/oder Beschädigungsgrad einer Bildplatte 1. Dieser Eintrag kann beispielsweise bei Systemen entfallen, in welchen die Bildplatte während des Auslesens auf einer festen, ebenen Unterlage aufliegt und folglich nicht gebogen wird.

Eine Steuerungs-Datengruppe IPC umfasst alle für eine Röntgenaufnahme spezifischen Daten, die für das Auslesen der Bildplatte 1 in der Auslesevorrichtung 20 erforderlich sind. Die Auslesevorrichtung 20 greift dabei ausschließlich lesend auf die in der Steuerungs-Datengruppe IPC gespeicherten Daten zu. Neben der Versionsnummer VN und der Prüfsumme CS umfasst die Steuerungs-Datengruppe IPC folgende Daten:

- Scannerempfindlichkeit: gibt die im Scanner 21 beim Auslesen der Bildplatte 1 einzustellende Empfindlichkeit an und ist abhängig von der Röntgendosis in Bereichen der Bildplatte 1, welche diagnostische Informationen enthalten;
- Röntgendosis: entspricht der maximalen Röntgendosis während einer Röntgenaufnahme und stellt ein Maß für die einzustellende Intensität der Löscheinrichtung 23 beim Löschen der Bildplatte 1 dar;
- Betriebsmodus: z.B. Weglassen oder Auslesen von Röntgeninformation aus bestimmten Randbereichen der Bildplatte 1.

In einer Verarbeitungs-Datengruppe IPP sind Daten gespeichert, die für eine Weiterverarbeitung von aus der Röntgeninformation der Bildplatte 1 gewonnenen Bilddaten erforderlich sind. Auf diese Daten wird ausschließlich von der Wiedergabevorrichtung 30, welche einen entsprechenden Bildprozessor zur Weiterverarbeitung der Bilddaten umfasst, zugegriffen. Der Auslesevorgang in der Auslesevorrichtung 20 wird durch diese Daten nicht beeinflusst.

Vorzugsweise werden die Daten der Verarbeitungs-Datengruppe IPP mit der zweiten Schreib-Lese-Einrichtung 24 in der Auslesevorrichtung 20 gelesen und zusammen mit den von der Bildplatte 1 gewonnenen Bilddaten im Zentralspeicher 40 abgelegt. Die Wiedergabevorrichtung 30 kann dann auf einfache Weise sowohl auf die zu verarbeitenden Bilddaten als auch auf die hierfür erforderlichen Daten der Verarbeitungs-Datengruppe IPP zugreifen.

In einer bevorzugten Ausgestaltung ist vorgesehen, die für die Weiterverarbeitung der Bilddaten erforderlichen Daten der Verarbeitungs-Datengruppe IPP in der ID-Station 10 einzugeben und diesen eindeutige Referenzcodes (IPP-Ref) zuzuordnen, die dann anstelle der Daten für die Weiterverarbeitung in den Speicher M geschrieben werden. Die Daten für die Weiterverarbeitung selbst werden zusammen mit den Referenzcodes (IPP-Ref) im Zentralspeicher 40 gespeichert. Die Wiedergabevorrichtung 30, welche die im Zentralspeicher 40 gespeicherten Daten für die Weiterverarbeitung benötigt, kann dann anhand der mit den Bilddaten von der Auslesevorrichtung 20 übertragenen Referenzcodes (IPP-Ref) auf die in der im Zentralspeicher 40 gespeicherten Daten für die Weiterverarbeitung zugreifen. Neben der Versionsnummer VN und der Prüfsumme CS umfasst die Verarbeitungs-Datengruppe IPP in diesem Fall folgende Daten:

- Referenzcode für die Art der durchzuführenden Weiterverarbeitung;

- Referenzcode für Aufnahme- und Patientendaten;
- Eindeutige Kennzeichnung des Zentralspeichers, auf welchen die Referenzcodes verweisen, wie z.B. der Name des Zentralspeichers in einem Netzwerk.

Eine Kalibrierungs-Datengruppe CAL umfasst Kalibrierdaten der Bildplatte 1. Die Kalibrierdaten liegen vorzugsweise als zweidimensionales Datenfeld vor, welches die unterschiedliche lokale Empfindlichkeit der Bildplatte 1 für Röntgenstrahlung widerspiegelt. Die Daten der Kalibrierungs-Datengruppe CAL werden vorzugsweise zusammen mit den Daten der Bildträger-Datengruppe IPI bei der Herstellung und Initialisierung der Bildplatte 1 bzw. der Kassette 2 im Speicher M des integrierten Schaltkreises 3 gespeichert und während der Bearbeitung der Bildplatte 1 bzw. der Kassette 2 nicht geändert. Neben der Versionsnummer VN und der Prüfsumme CS umfasst die Kalibrierungs-Datengruppe CAL im Einzelnen folgende Daten:

- Anzahl der Spalten des zweidimensionalen Datenfeldes der Kalibrierdaten;
- Anzahl der Zeilen des zweidimensionalen Datenfeldes der Kalibrierdaten;
- zweidimensionales Datenfeld der Kalibrierdaten.

Die Kalibrierdaten der Bildplatte 1 werden in der Auslesevorrichtung 20 zu einer Vorauswertung der beim Auslesen der Bildplatte 1 mittels Scanner 21 erhaltenen Bildsignale herangezogen, um lokal unterschiedliche Empfindlichkeiten der Bildplatte 1 für Röntgenstrahlung zu berücksichtigen. Die Kalibrierdaten werden hierzu in der Auslesevorrichtung 20 aus dem integrierten Schaltkreis 3 gelesen und der Vorverarbeitung der Bildsignale zugeführt.

Vorzugsweise werden einmal ausgelesene Kalibrierdaten in einem Speicher (nicht dargestellt) der Auslesevorrichtung 20 gespeichert oder über den ersten Datenbus 25 an den Zentralspeicher 40 übertragen und dort gespeichert. Wird dieselbe Bildplatte 1 mit einer anderen Röntgenaufnahme dann erneut einem Auslesevorgang in derselben Auslesevorrichtung 20 unterzogen, so kann diese Auslesevorrichtung 20 direkt auf den Speicher der Auslesevorrichtung 20 bzw. den Zentralspeicher 40 zugreifen und die für die Vorverarbeitung der Bildsignale erforderlichen Kalibrierdaten der Bildplatte 1 abrufen. Die Kalibrierdaten stehen dadurch in kürzerer Zeit für die Vorverarbeitung zur Verfügung als bei einem erneuten Auslesen des zweidimensionalen Datensatzes aus dem integrierten Schaltkreis 3. Auch etwaige Übertragungsfehler beim Auslesen des Datensatzes aus dem integrierten Schaltkreis können dadurch vermieden werden.

Alternativ können die Kalibrierdaten bereits an der ID-Station 10 aus dem integrierten Schaltkreis 3 ausgelesen, über einen zweiten Datenbus 17 an den Zentralspeicher 40 übertragen und dort gespeichert werden. Ein Auslesen und Übertragen der Kalibrierdaten zum Zentralspeicher 40 während des ersten Auslesevorgangs in der Auslesevorrichtung 20 kann dann entfallen. Der zweite Datenbus 17 ist vorzugsweise ebenfalls als serieller Datenbus, z.B. als RS-232-Datenbus, ausgebildet.

Prinzipiell ist es möglich, das zweidimensionale Datenfeld der Kalibrierdaten der Bildplatte 1 bei jedem Auslesevorgang in der Auslesevorrichtung 20 aus dem integrierten Schaltkreis 3 von Neuem auszulesen. In diesem Fall kann gegebenenfalls auf eine permanente Verbindung zwischen der Auslesevorrichtung 20 und dem Zentralspeicher 40 ganz verzichtet werden oder zumindest der Datenfluss zwischen den einzelnen Komponenten des Systems, insbesondere zwischen Auslesevorrichtung 20 und Zentralspeicher 40 besonders niedrig gehalten werden.

Neben den Kalibrierdaten der Bildplatte 1 werden im Zentralspeicher 40 oder im Speicher der Auslesevorrichtung 20 auch Kalibrierdaten des Scanners 21 abgelegt, welche lokale Unterschiede in der Empfindlichkeit des Scanners 21 widerspiegeln und vorzugsweise zusammen mit den Kalibrierdaten der Bildplatte 1 zur Vorverarbeitung der Bildsignale herangezogen werden. Aus der Vorverarbeitung der Bildsignale werden schließlich Bilddaten des in der Bildplatte 1 gespeicherten Röntgenbildes erhalten, aus welchen der Einfluss lokal unterschiedlicher Empfindlichkeiten der Bildplatte 1 und des Scanners 21 eliminiert worden ist.

Typischerweise weist das zweidimensionale Datenfeld der Kalibrierdaten der Bildplatte 1 zwischen etwa 30 und 40 Spalten und zwischen etwa 40 und 50 Zeilen auf, wobei die Länge der jeweiligen Daten jeweils zwei Bytes beträgt. Die für ein solches zweidimensionales Datenfeld zu reservierende Speichergröße beträgt demnach für ein Datenfeld, welches vorzugsweise 35 Spalten und 43 Zeilen aufweist, 2 x 35 x 43 = 3010 Bytes.

In dem beschriebenen Beispiel geben die Kalibrierdaten die Empfindlichkeit von einzelnen Bereichen der Speicherleuchtstoff-Schicht 5 wieder, die größer sind als die einzelnen, beim zeilenweisen Auslesen der Bildplatte 1 erhaltenen Bildpunkte (Pixel). Auf diese Weise kann der Speicherplatzbedarf für die Kalibrierdaten im integrierten Schaltkreis 3 stark reduziert werden, wobei gleichzeitig lokal unterschiedliche Empfindlichkeiten mit einer hohen Genauigkeit berücksichtigt werden.

Wenn ausreichend Speicherplatz im integrierten Schaltkreis 3 vorhanden ist, können die Kalibrierdaten aber auch die Empfindlichkeit der Bildplatte 1 in einzelnen Bereichen der Bildplatte wiedergeben, die den einzelnen Bildpunkten entsprechen. Hierdurch wird eine pixelgenaue Kalibrierung ermöglicht, die lokal unterschiedliche Empfindlichkeiten mit noch höherer Genauigkeit berücksichtigt.

Um möglichst wenig Speicherplatz im Speicher M des integrierten Schaltkreises 3 zu beanspruchen, werden die Kalibrierdaten der Bildplatte 1 vorteilhafterweise einem Komprimierungsverfahren unterzogen, bevor diese im integrierten Schaltkreis 3 abgelegt werden.

In einer Patienten-Datengruppe IDP sind Da-

ten gespeichert, welche für einen zu untersuchenden Patienten spezifisch sind. Neben der Versionsnummer VN und der Prüfsumme CS umfasst die Patienten-Datengruppe IDP beispielsweise folgende Daten:

- Name des Patienten;
- Geburtsdatum des Patienten;
- Geschlecht des Patienten.

Vorzugsweise wird anstelle der oder zusätzlich zu den genannten patientenspezifischen Daten der Patienten-Datengruppe IDP ein Referenzcode IDP-Ref in den Speicher M geschrieben, welcher eine Referenz auf die Daten der Patienten-Datengruppe IDP darstellt, die im Zentralspeicher 40 abgelegt sind. Der Referenzcode IDP-Ref, welcher auch als Patienten-Identifikationscode bezeichnet wird und eine eindeutige Identifikation des Patienten ermöglicht, wird mit der ersten Schreib-Lese-Einrichtung 11 der ID-Station 10 in den Speicher M des integrierten Schaltkreises 3 geschrieben und zusammen mit den Daten der Patienten-Datengruppe IDP im Zwischenspeicher 16 der ID-Station 10 und/oder im Zentralspeicher 40 gespeichert.

Anhand des Patienten-Identifikationscodes IDP-Ref kann dann auf die im Zwischenspeicher 16 bzw. Zentralspeicher 40 gespeicherten patientenspezifischen Daten zugegriffen werden. Der Patienten-identifikationscode IDP-Ref wird hierzu entweder in der ID-Station 10 bzw. in der Auslesevorrichtung 20 aus dem integrierten Schaltkreis 3 gelesen und an die Wiedergabevorrichtung 30 übertragen oder zusammen mit den aus der Bildplatte 1 ausgelesenen Bilddaten an die Wiedergabevorrichtung 30 übertragen, welche dann anhand des Patienten-Identifikationscodes IDP-Ref auf die entsprechenden patientenspezifischen Daten im Zwischenspeicher 16 bzw. Zentralspeicher 40 zugreifen kann.

Prinzipiell kann die Patienten-Datengruppe IDP aber auch entfallen, wenn in der Verarbeitungs-Datengruppe IPP - wie oben bereits beschrieben - bereits Referenzcodes für Patientendaten vorgesehen sind, welche u.a. auf die entsprechenden Patientendaten, wie z.B. Name und/oder Geburtsdatum und/oder Geschlecht des Patienten, im Zentralspeicher 40 verweisen.

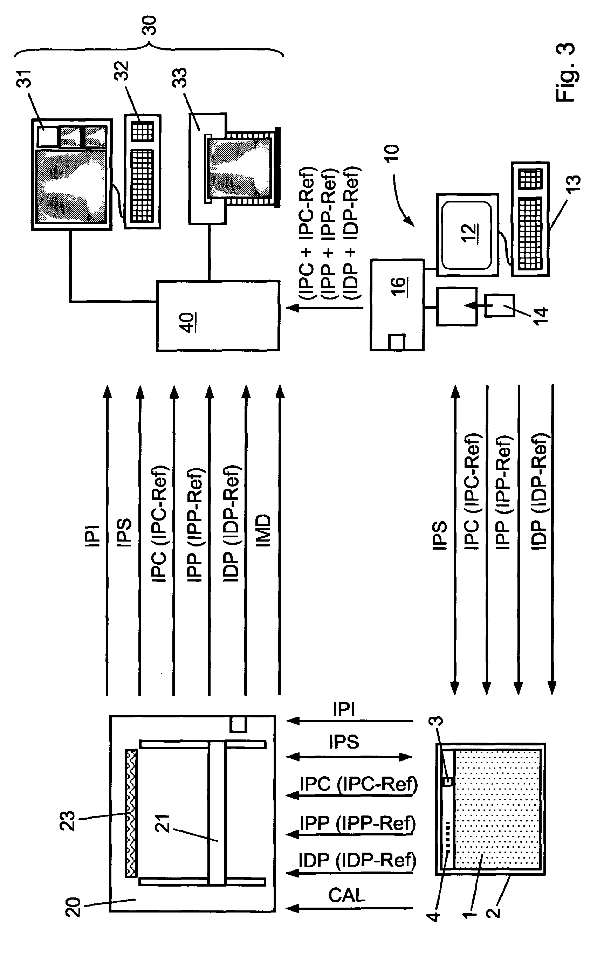

Fig. 3 zeigt eine schematische Darstellung einer ersten Variante des Datenflusses zwischen den einzelnen Komponenten des in Fig. 1 dargestellten erfindungsgemäßen Systems. Der Datenfluss wird nachfolgend anhand eines kompletten Radiographievorgangs näher erläutert.

Unmittelbar nach einer Röntgenaufnahme eines Patienten wird die Kassette 2 mit der darin befindlichen Bildplatte 1 zur ID-Station 10 gebracht. An der ID-Station 10 werden folgende Schritte durchgeführt:

    a) Lesen von Daten der Zustands-Datengruppe IPS zum Bearbeitungsstatus der Bildplatte 1 aus dem Speicher M des integrierten Schaltkreises 3. Prüfen, ob der Bearbeitungsstatus auf "Bildplatte initialisiert" oder "Bildplatte gelöscht" gesetzt ist. Ist die Bildplatte nicht initialisiert oder nicht gelöscht, muss der Bediener zwischen zwei Alternativen wählen, nämlich einem Überschreiben der Daten oder einem Abbruch der Bearbeitung.

    b) Eingabe von patientenspezifischen Daten der Patienten-Datengruppe IDP mittels Tastatur 13 und/oder Anzeigeeinheit 12 und/oder Karte 14.
    c) Eingabe von für das Auslesen der Bildplatte 1 in der Auslesevorrichtung 20 erforderlichen Daten der Steuerungs-Datengruppe IPC mittels Tastatur 13 und/oder Anzeigeeinheit 12.
    d) Eingabe von für die Weiterverarbeitung von Bilddaten in der Wiedergabevorrichtung 30 erforderlichen Daten der Verarbeitungs-Datengruppe IPP mittels Tastatur 13 und/oder Anzeigeeinheit 12.
    e) Schreiben der eingegebenen Daten der Steuerungs-, Verarbeitungs- und Patienten-Datengruppe IPC, IPP bzw. IDP in den Speicher M des integrierten Schaltkreises 3 der Bildplatte 1 bzw. Kassette 2.
    f) Schreiben von Daten der Zustands-Datengruppe IPS zum aktuellen Bearbeitungsstatus in den Speicher M: Bearbeitungsstatus wird auf "Röntgenbild aufgenommen" gesetzt.

Anschließend wird die Kassette 2 mit der darin befindlichen Bildplatte 1 zur Auslesevorrichtung 20 gebracht. Dort wird die Bildplatte 1 mit dem Scanner 21 zeilenweise ausgelesen, wobei Bildsignale erzeugt werden, die den in der Bildplatte 1 gespeicherten Röntgeninformationen entsprechen. Die Bildsignale werden einer Vorverarbeitung unterzogen, bei der Bilddaten IMD erhalten werden, die an die Wiedergabevorrichtung 30 weitergegeben werden können. In der Auslesevorrichtung 20 werden dabei im Einzelnen folgende Schritte durchgeführt:

    a) Lesen von Daten der Bildträger-, Zustands-, Steuerungs-, Verarbeitungs- und Patienten-Datengruppe IPI, IPS, IPC, IPP bzw. IDP aus dem Speicher M.
    b) Lesen von Daten der Kalibrierungs-Datengruppe CAL, d.h. von Kalibrierdaten der Bildplatte 1, aus dem Speicher M.
    Oder:

        Lesen von bereits bei einem früheren Auslesevorgang dieser Bildplatte 1 in einem Speicher (nicht dargestellt) der Auslesevorrichtung 20 gespeicherten Daten der Kalibrierungs-Datengruppe CAL.

    c) Prüfen der gelesenen Daten der Zustands-Datengruppe IPS, ob der Bearbeitungsstatus der Bildplatte 1 auf "Röntgenbild aufgenommen" gesetzt ist. Andernfalls wird die weitere Bearbeitung abgebrochen.
    d) Lesen von in einem Speicher der Auslesevorrichtung 20 gespeicherten Kalibrierdaten (CALS) des Scanners 21.

e) Zeilenweises Auslesen der in der Bildplatte 1 gespeicherten Röntgeninformationen mit dem Scanner 21 unter Heranziehung von Daten der Bildträger- und Steuerungs-Datengruppe IPI bzw. IPC und Erzeugen von entsprechenden Bildsignalen.

f) Vorverarbeitung der erzeugten Bildsignale anhand der Kalibrierdaten CAL der Bildplatte 1 und der Kalibrierdaten (CALS) des Scanners 21 zu Bilddaten IMD.

g) Löschen von etwaigen restlichen Röntgeninformationen in der Bildplatte 1 mit der Löscheinrichtung 23 unter Heranziehung von Daten der Bildträger- und Steuerungs-Datengruppe IPI bzw. IPC.

h) Speichern der Bilddaten IMD zusammen mit den Daten der Verarbeitungsund Patienten-Datengruppe IPP bzw. IDP im Zentralspeicher 40.

i) Schreiben von Daten der Zustands-Datengruppe IPS zum aktuellen Bearbeitungsstatus der Bildplatte 1 in den Speicher M: "Bildplatte ausgelesen" bzw. "Bildplatte gelöscht".

Optional können in Schritt h) zusätzlich die Daten der Bildtärger- und/oder Steuerungs-Datengruppe IPI bzw. IPC zusammen mit den Bilddaten IMD im Zentralspeicher 40 gespeichert werden.

Die beim Auslesen der Bildplatte 1 erzeugten Bilddaten IMD können dann in der Wiedergabevorrichtung 30 auf einem Monitor 31 dargestellt und/oder auf einem Hardcopygerät 33 ausgegeben werden. Im Einzelnen werden hierbei folgende Schritte durchgeführt:

a) Lesen der Bilddaten IMD und der zugehörigen Daten der Verarbeitungs- und Patienten-Datengruppe IPP bzw. IDP aus dem Zentralspeicher 40.

b) Weiterverarbeitung der Bilddaten IMD auf der Grundlage der Daten der Verarbeitungs-Datengruppe IPP.

c) Wiedergabe - z.B. am Monitor 31 und/oder Hardcopygerät 33 - der weiterverarbeiteten Bilddaten IMD unter Heranziehung der Daten der Verarbeitungsund/oder Patienten-Datengruppe IPP bzw. IDP.

In einer Abwandlung des in Fig. 3 dargestellten Datenflusses werden an der ID-Station 10 anstelle der Daten der Steuerungs-, Verarbeitungs- und Patienten-Datengruppe IPC, IPP bzw. IDP ein oder mehrere Referenzcodes IPC-Ref, IPP-Ref bzw. IDP-Ref in den Speicher M des integrierten Schaltkreises 3 der Bildplatte 1 bzw. Kassette 2 geschrieben. Die Referenzcodes IPC-Ref, IPP-Ref bzw. IDP-Ref werden zusammen mit den entsprechenden Daten der Steuerungs-, Verarbeitungs- und Patienten-Datengruppe IPC, IPP bzw. IDP im Zentralspeicher 40 gespeichert. Anhand der Referezcodes IPC-Ref, IPP-Ref bzw. IDP-Ref kann dann die Auslesevorrichtung 20 auf die im Zentralspeicher 40 gespeicherten Daten der entsprechenden Datengruppe IPC, IPP bzw. IDP zugreifen. Die bei dieser Abwandlung übertragenen Referenzcodes IPC-Ref, IPP-Ref bzw. IDP-Ref, ggf. mit den zugehörigen Daten der entsprechenden Datengruppe IPC, IPP bzw. IDP sind in der Fig. 3 in runde Klammern () gesetzt. Im übrigen gelten die obigen Ausführungen entsprechend.

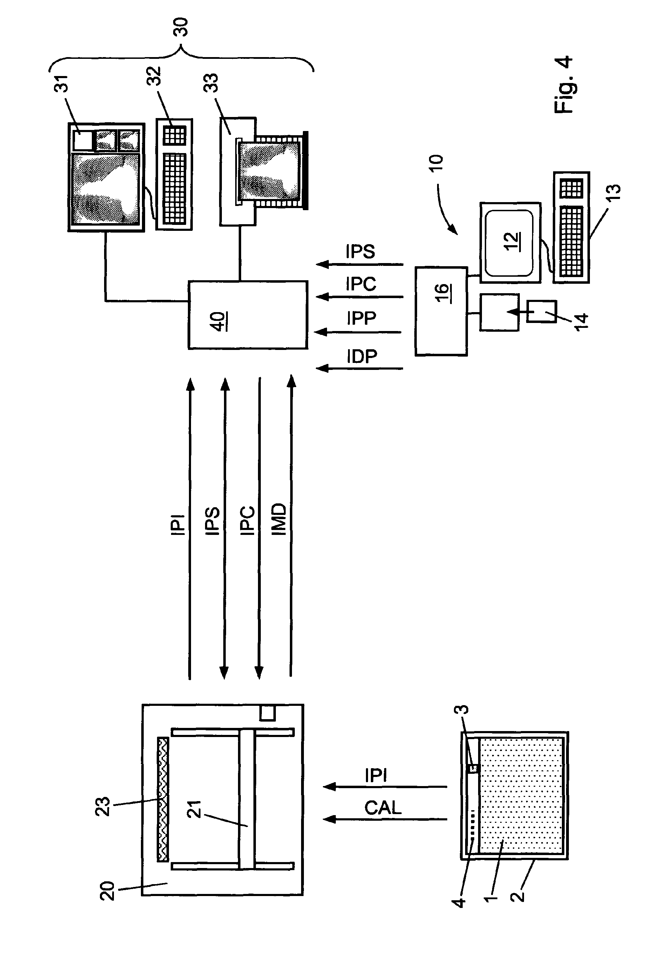

Fig. 4 zeigt eine schematische Darstellung einer zweiten Variante des Datenflusses zwischen den einzelnen Komponenten des in Fig. 1 dargestellten Systems. Bei dieser Variante des Datenflusses werden an der ID-Station 10 -wie oben näher beschrieben - Daten der Zustands-, Steuerungs-, Verarbeitungs- und Patienten-Datengruppe IPS, IPC, IPP bzw. IDP eingegeben, jedoch nicht in den Speicher M des auf der Bildplatte 1 befindlichen integrierten Schaltkreises 3 geschrieben, sondern an den Zentralspeicher 40 übertragen und dort gespeichert. Im Speicher M des integrierten Schaltkreises 3 sind bei dieser Variante lediglich Daten der Bildträger- und Kalibrierungs-Datengruppe IPI bzw. CAL gespeichert. Entsprechend werden in der Auslesevorrichtung 20 lediglich Daten der Bildträgerund Kalibrierungs-Datengruppe IPI bzw. CAL, welche bildplattenspezifische Daten bzw. die Kalibrierdaten der Bildplatte 1 umfassen, aus dem integrierten Schaltkreis 3 gelesen. Die Daten der Zustands-Datengruppe IPS zum Bearbeitungsstatus der Bildplatte 1 sowie die für das Auslesen der Röntgeninformation aus der Bildplatte 1 sowie das anschließende Löschen restlicher Bildinformation erforderlichen Daten der Steuerungs-Datengruppe IPC werden dagegen aus dem Zentralspeicher 40 gelesen. Im Übrigen gelten die Ausführungen zu Fig. 3 entsprechend.

Bei den in den Beispielen der Figuren 3 und 4 gezeigten Bildplatten 1 ist die Markierung 4, die einen Teil der im Speicher M des integrierten Schaltkreises 3 gespeicherten Daten, insbesondere Daten der Bildträger-Datengruppe IPI, repräsentiert, auf der Rückseite der Trägerschicht der Bildplatte 1 angebracht. Die Markierung 4 ist daher gestrichelt dargestellt.

Fig. 5 zeigt ein zweites Beispiel einer Struktur der im integrierten Schaltkreis 3 des Bildträgers gespeicherten Daten in der in Fig. 4 dargestellten Variante des Datenflusses. Wie bereits erläutert, sind bei dieser Variante lediglich Daten der Bildträger- und Kalibrierungs-Datengruppe IPI bzw. CAL im Speicher M gespeichert. Für die Struktur und den Inhalt der einzelnen Datengruppen IPI bzw. CAL gelten die obigen Ausführungen zu Fig. 2 entsprechend.

Bei dieser Variante kann der Speicherplatzbedarf im Speicher M des integrierten Schaltkreises gegenüber dem in den Figuren 2 und 3 gezeigten Beispiel reduziert werden.

Bei dem in den Figuren 2 und 3 gezeigten Beispiel dagegen wird mehr Speicherplatz benötigt, wobei jedoch die Auslesevorrichtung 20 die für das Auslesen bzw. Löschen erforderlichen Daten direkt aus dem Speicher M des integrierten Schaltkreises 3 auf der Bildplatte entnehmen kann und hierfür nicht auf den Zentralspeicher 40 zugreifen muss. Dadurch wird eine Unabhängigkeit der Auslesevorrichtung 20 von einem Zentralspeicher 40 erreicht.

**Patentansprüche**

1. Verfahren zur Bearbeitung eines Bildträgers (1, 2) zur Aufzeichnung von Röntgenaufnahmen, welcher eine Bildplatte (1) zur Aufzeichnung der Röntgenaufnahme sowie einen elektronischen Speicher (M) zur Speicherung von Daten umfasst, **dadurch gekennzeichnet, dass** ein erster elektronischer Speicher (M) bei einem Defekt durch einen zweiten elektronischen Speicher ersetzt wird und die in einer Markierung (4) auf dem Bildträger (1, 2) enthaltenen Daten, welche zumindest einen Teil der im ersten elektronischen Speicher (M) gespeicherten Daten zu Eigenschaften des Bildträgers (1, 2) repräsentieren, in den zweiten elektronischen Speicher geschrieben werden, wobei die im ersten elektronischen Speicher (M) gespeicherten Daten zu Eigenschaften des Bildträgers (1, 2) eines oder mehrere der folgenden Daten umfassen:

   - Größe einer auszulesenden Fläche auf dem Bildträger (1);
   - Empfindlichkeit des Bildträgers (1);
   - Löscheigenschaft des Bildträgers (1), insbesondere als Maß für die Dauer und/oder Intensität von von einer Löscheinrichtung abgegebenem Licht.

2. Verfahren nach Anspruch 1, **dadurch gekennzeichnet, dass** die Markierung (4) die Daten in Klarschrift oder maschinenlesbarer Form wiedergibt.

3. Verfahren nach Anspruch 2 **dadurch gekennzeichnet, dass** die Markierung (4) eine alphanumerische Zeichenfolge umfasst.

4. Verfahren nach einem der vorangehenden Ansprüche, **dadurch gekennzeichnet, dass** die Daten im elektronischen Speicher (M) in unterschiedlichen Datengruppen (IPI, IPS, IPC, IPP, CAL, IDP) gespeichert sind.

5. Verfahren nach Anspruch 4, **dadurch gekennzeichnet, dass** die Daten mindestens einer Datengruppe (IPI, IPS, IPC, IPP, CAL, IDP) eine Versionsnummer (VN) umfassen, welche die Struktur, in welcher die Daten dieser Datengruppe (IPI, IPS, IPC, IPP, CAL, IDP) vorliegen, kennzeichnet.

6. Verfahren nach einem der Ansprüche 4 oder 5, **dadurch gekennzeichnet, dass** mindestens eine Datengruppe (IPI, IPS, IPC, IPP, CAL, IDP) eine aus den Daten dieser Datengruppe (IPI, IPS, IPC, IPP, CAL, IDP) abgeleitete Prüfsumme (CS) umfasst, anhand welcher überprüft werden kann, ob die Daten dieser Datengruppe (IPI, IPS, IPC, IPP, CAL, IDP) fehlerfrei gespeichert und/oder gelesen worden sind.

7. System zur Bearbeitung eines Bildträgers (1, 2) zur Aufzeichnung von Röntgenaufnahmen mit

   - einem Bildträger (1, 2), welcher eine Bildplatte (1) zur Aufzeichnung der Röntgenaufnahme sowie einen elektronischen Speicher (M) zur Speicherung von Daten umfasst, und
   - einer ID-Station (10), an welcher Daten eingegeben und in den elektronischen Speicher (M) geschrieben werden können,

   **dadurch gekennzeichnet, dass**
   der elektronische Speicher (M) lösbar am Bildträger (1, 2) befestigt ist, so dass ein erster elektronischer Speicher (M) bei einem Defekt durch einen zweiten elektronischen Speicher ersetzt werden kann, und dann die ID-Station (10) die in einer Markierung (4) auf dem Bildträger (1, 2) enthaltenen Daten, welche zumindest einen Teil der im ersten elektronischen Speicher (M) gespeicherten Daten zu Eigenschaften des Bildträgers (1, 2) repräsentieren, in den zweiten elektronischen Speicher (M) schreibt, wobei die im ersten elektronischen Speicher (M) gespeicherten Daten zu Eigenschaften des Bildträgers (1, 2) eines oder mehrere der folgenden Daten umfassen:

   - Größe einer auszulesenden Fläche auf dem Bildträger (1);
   - Empfindlichkeit des Bildträgers (1);
   - Löscheigenschaft des Bildträgers (1), insbesondere als Maß für die Dauer und/oder Intensität von von einer Löscheinrichtung abgegebenem Licht.

8. System nach Anspruch 7, **dadurch gekennzeichnet, dass** die ID-Station (10) eine Tastatur (13) umfasst, über welche die durch die Markierung (4) repräsentierten Daten eingegeben werden können.

9. System nach Anspruch 8, **dadurch gekennzeichnet, dass** die ID-Station (10) ein Lesegerät umfasst, über welches die durch die Markierung (4) repräsentierten Daten eingelesen werden können.

10. System nach einem der Ansprüche 7 bis 9, **dadurch gekennzeichnet, dass** die ID-Station (10) eine Schreib-Lese-Einrichtung (11), insbesondere einen RF-Sender und einen RF-Empfänger, umfasst, welche zur kontaktlosen Übertragung von Daten zwischen der ID-Station (10) und dem elektronischen Speicher (M) ausgebildet ist.

11. System nach einem der Ansprüche 7 bis 10, **da-**

**durch gekennzeichnet, dass** die Markierung (4) an der Bildplatte (1) angebracht ist.

12. System nach Anspruch 11, **dadurch gekennzeichnet, dass** die Bildplatte (1) eine Trägerschicht mit einer auf der Vorderseite der Trägerschicht befindlichen Speicherleuchtstoff-Schicht (5) umfasst und die Markierung (4) in einem Randbereich der Trägerschicht, insbesondere außerhalb der Speicherleuchtstoff-Schicht (5), und/oder an der der Vorderseite gegenüberliegenden Rückseite der Trägerschicht angebracht ist.

13. System nach einem der Ansprüche 7 bis 10, **dadurch gekennzeichnet, dass** der Bildträger (1, 2) eine Kassette (2) umfasst, welche die Bildplatte (1) aufnehmen kann.

14. System nach Anspruch 13, **dadurch gekennzeichnet, dass** die Markierung (4) an der Kassette (2) angebracht ist.

15. System nach einem der Ansprüche 7 bis 14, **dadurch gekennzeichnet, dass** der elektronische Speicher (M) zur kontaktlosen Übertragung von Daten von und zu mindestens einer Bearbeitungsvorrichtung (10, 20) ausgebildet ist.

16. System nach Anspruch 15, **dadurch gekennzeichnet, dass** der elektronische Speicher (M) als RF-Etikett ausgebildet ist, das den elektronischen Speicher (M) und eine Antenne, insbesondere eine Transponderspule, umfasst.

17. System nach Anspruch 15, **dadurch gekennzeichnet, dass** der elektronische Speicher (M) als Chipkarte ausgebildet ist, die einen Kartenkörper umfasst, in welchen der elektronische Speicher (M) und eine Antenne, insbesondere eine Transponderspule, eingebracht sind.

18. System nach Anspruch 17 sowie Anspruch 7 oder Anspruch 13, **dadurch gekennzeichnet, dass** die Chipkarte lösbar an der Bildplatte (1) bzw. Kassette (2) befestigt ist.

19. System nach Anspruch 18, **dadurch gekennzeichnet, dass** die Chipkarte mittels einer Steckverbindung an der Bildplatte (1) bzw. Kassette (2) befestigt ist.

20. System nach Anspruch 19, **dadurch gekennzeichnet, dass** die Steckverbindung einen Mechanismus, insbesondere einen Schnappmechanismus, zum Arretieren der Chipkarte in der Steckverbindung aufweist.

**Claims**

1. A method of working with an image carrier (1, 2) for recording radiograms which comprises an image plate (1) for recording the radiogram and an electronic memory (M) for storing data, **characterised in that** with a defect a first electronic memory (M) is replaced by a second electronic memory, and the data included in a mark (4) on the image carrier (1, 2) which represent at least part of the data on properties of the image carrier (1, 2) stored in the first electronic memory (M) are written into the second electronic memory, the data on properties of the image carrier (1,2) stored in the first electronic memory (M) comprising one or more of the following data:

   - size of an area on the image carrier (1) to be read out;
   - sensitivity of the image carrier (1);
   - deletion property of the image carrier (1), in particular as a measure of the duration and/or intensity of light emitted by a deletion device.

2. The method according to Claim 1, **characterised in that** the mark (4) reproduces the data in plain writing or in machine-readable form.

3. The method according to Claim 2, **characterised in that** the mark (4) comprises an alphanumerical string of characters.

4. The method according to any of the preceding claims, **characterised in that** the data are stored in the electronic memory (M) in different data groups (IPI, IPS, IPC, IPP CAL, IDP).

5. The method according to Claim 4, **characterised in that** the data of at least one data group (IPI, IPS, IPC, IPP, CAL, IDP) comprise a version number (VN) which identifies the structure in which the data of this data group (IPI, IPS, IPC, IPP, CAL, IDP) are provided.

6. The method according to any of Claims 4 or 5, **characterised in that** at least one data group (IPI, IPS, IPC, IPP, CAL, IDP) comprises a check sum (CS) derived from the data of this data group (IPI, IPS, IPC, IPP, CAL, IDP) by means of which it can be checked whether the data of this data group (IPI, IPS, IPC, IPP, CAL, IDP) have been stored error-free and/or have been read.

7. A system for working with an image carrier (1, 2) for recording radiograms with

   - an image carrier (1, 2) which comprises an image plate (1) for recording the radiogram and an electronic memory (M) for storing data, and

- an ID station (10) where data can be inputted and written into the electronic memory (M),

**characterised in that**

- the electronic memory (M) is attached releaseably to the image carrier (1, 2) so that with a defect a first electronic memory (M) can be replaced by a second electronic memory, and then the ID station (10) writes the data contained in a marking (4) on the image carrier (1, 2) which represent at least part of the data on the properties of the image carrier (1, 2) stored in the first electronic memory (M) into the second electronic memory (M), the data on properties of the image carrier (1, 2) stored in the first electronic memory (M) comprising one or more of the following data:

- size of an area on the image carrier (1) to be read out;
- sensitivity of the image carrier (1);
- deletion property of the image carrier (1), in particular as measure of the duration and/or the intensity of light emitted by a deletion device.

8. The system according to Claim 7, **characterised in that** the ID station (10) comprises a key pad (13) by means of which the data represented by the mark (4) can be inputted.

9. The system according to Claim 8, **characterised in that** the ID station (10) comprises a reader by means of which the data represented by the mark (4) can be read in.

10. The system according to any of Claims 7 to 9, **characterised in that** the ID station (10) comprises a writing/reading device (11), in particular a RF transmitter and a RF receiver which is designed for the contact-free transmission of data between the ID station (10) and the electronic memory (M).

11. The system according to any of Claims 7 to 10, **characterised in that** the mark (4) is applied to the image plate (1).

12. The system according to Claim 11, **characterised in that** the image plate (1) comprises a carrier layer with a storage phosphor layer (5) located on the front side of the carrier layer and the mark (4) is applied to a peripheral region of the carrier layer, in particular outside of the storage phosphor layer (5) and/or to the rear side of the carrier layer opposite the front side.

13. The system according to any of Claims 7 to 10, **char-**acterised in that** the image carrier (1, 2) comprises a cassette (2) which can accommodate the image plate (1).

14. The system according to Claim 13, **characterised in that** the mark (4) is applied to the cassette (2).

15. The system according to any of Claims 7 to 14, **characterised in that** the electronic memory (M) is designed for the contact-free transmission of data from and to at least one processing apparatus (10, 20).

16. The system according to Claim 15, **characterised in that** the electronic memory (M) is in the form of an RF marker which comprises the electronic memory (M) and an antenna, in particular a transponder coil.

17. The system according to Claim 15, **characterised in that** the electronic memory (M) is in the form of a chip card which comprises a card body into which the electronic memory (M) and an antenna, in particular a transponder coil, is inserted.

18. The system according to Claim 17 and Claim 7 or Claim 13, **characterised in that** the chip card is attached releaseably to the image plate (1) or cassette (2).

19. The system according to Claim 18, **characterised in that** the chip card is attached to the image plate (1) or cassette (2) by means of a plug connection.

20. The system according to Claim 19, **characterised in that** the plug connection has a mechanism, in particular a snap mechanism, for locking the chip card in the plug connection.

**Revendications**

1. Procédé de traitement d'un support d'image (1, 2) pour l'enregistrement de radiographies, comprenant un vidéodisque (1) pour la mémorisation de la radiographie ainsi qu'une mémoire électronique (M) pour la mémorisation de données, **caractérisé en ce qu'**une première mémoire électronique (M) est remplacée par une seconde mémoire électronique en cas de défaillance et **en ce que** les données qui sont contenues dans le marquage (4) présent sur le support d'image (1, 2) et qui représentent au moins une partie des données mémorisées dans la première mémoire électronique (M) concernant les propriétés du support d'image (1, 2) sont écrites dans la deuxième mémoire électronique, les données mémorisées dans la première mémoire électronique (M) concernant les propriétés du support d'image (1, 2) comprenant une ou plusieurs des données suivantes :

- taille d'une surface destinée à être lue sur le support d'image (1) ;
- sensibilité du support d'image (1) ;
- caractéristiques d'effacement du support d'image (1), indicatives notamment de la durée et/ou de l'intensité de la lumière émise par un dispositif d'effacement.

2. Procédé selon la revendication 1, **caractérisé en ce que** le marquage (4) reproduit les données sous une forme lisible par machine ou à l'oeil nu.

3. Procédé selon la revendication 2, **caractérisé en ce que** le marquage (4) comprend une suite de caractères alphanumériques.

4. Procédé selon l'une des revendications précédentes, **caractérisé en ce que** les données de la mémoire électronique (M) sont mémorisées dans différents groupes de données (IPI, IPS, IPC, IPP, CAL, IDP).

5. Procédé selon la revendication 4, **caractérisé en ce que** les données d'au moins un groupe de données (IPI, IPS, IPC, IPP, CAL, IDP) comprennent un numéro de version (VN) qui caractérise la structure dans laquelle se trouvent les données de ce groupe de données (IPI, IPS, IPC, IPP, CAL, IDP).

6. Procédé selon l'une des revendications 4 ou 5, **caractérisé en ce qu'**au moins un groupe de données (IPI, IPS, IPC, IPP, CAL, IDP) comprend une somme de contrôle (CS) qui est dérivée des données de ce groupe de données (IPI, IPS, IPC, IPP, CAL, IDP) et à l'aide de laquelle on peut vérifier si les données de ce groupe de données (IPI, IPS, IPC, IPP, CAL, IDP) ont été mémorisées et/ou lues sans erreur.

7. Système de traitement d'un support d'image (1, 2) pour l'enregistrement de radiographies comprenant

- un support d'image (1, 2), comprenant un vidéodisque (1) pour la mémorisation de la radiographie ainsi qu'une mémoire électronique (M) pour la mémorisation de données, et
- un poste d'identification ID (10), au niveau duquel des données peuvent être saisies et être écrites dans la mémoire électronique (M),

**caractérisé en ce que**
la mémoire électronique (M) est fixée au support d'image (1, 2) de manière amovible de sorte qu'une première mémoire électronique (M) puisse être remplacée par une seconde mémoire électronique en cas de défaillance, puis que le poste d'identification ID (10) écrive dans la deuxième mémoire électronique les données qui sont contenues dans le marquage (4) présent sur le support d'image (1, 2) et qui représentent au moins une partie des données mémorisées dans la première mémoire électronique (M) concernant les propriétés du support d'image (1, 2), les données mémorisées dans la première mémoire électronique (M) concernant les propriétés du support d'image (1, 2) comprenant une ou plusieurs des données suivantes :

- taille d'une surface destinée à être lue sur le support d'image (1) ;
- sensibilité du support d'image (1) ;
- caractéristiques d'effacement du support d'image (1), indicatives notamment de la durée et/ou de l'intensité de la lumière émise par un dispositif d'effacement.

8. Système selon la revendication 7, **caractérisé en ce que** le poste d'identification ID (10) comprend un clavier (13) permettant de saisir les données représentées par le marquage (4).

9. Système selon la revendication 8, **caractérisé en ce que** le poste d'identification ID (10) comprend un appareil de lecture permettant de lire les données représentées par le marquage (4).

10. Système selon l'une des revendications 7 à 9, **caractérisé en ce que** le poste d'identification ID (10) comprend un dispositif d'écriture-lecture (11), notamment un émetteur RF et un récepteur RF, conçu pour la transmission sans contact de données entre le poste d'identification ID (10) et la mémoire électronique (M).

11. Système selon l'une des revendications 7 à 10, **caractérisé en ce que** le marquage (4) est appliqué sur le vidéodisque (1).

12. Système selon la revendication 11, **caractérisé en ce que** le vidéodisque (1) comprend une couche de support présentant une couche luminescente de mémorisation (5) sur sa face avant et **en ce que** le marquage (4) est appliqué dans une zone marginale de la couche de support, notamment en dehors de la couche luminescente de mémorisation (5), et/ou sur la face arrière opposée à la face avant de la couche de support.

13. Système selon l'une des revendications 7 à 10, **caractérisé en ce que** le support d'image (1, 2) comprend une cassette (2) susceptible de recevoir le vidéodisque (1).

14. Système selon la revendication 13, **caractérisé en ce que** le marquage (4) est appliqué sur la cassette (2).

15. Système selon l'une des revendications 7 à 14, **ca-**

**ractérisé en ce que** la mémoire électronique (M) est conçue pour la transmission sans contact de données en provenance et à destination d'au moins un dispositif de traitement (10, 20).

16. Système selon la revendication 15, **caractérisé en ce que** la mémoire électronique (M) est conçue sous la forme d'une étiquette RF qui comprend la mémoire électronique (M) et une antenne, notamment une bobine de transpondeur.

17. Système selon la revendication 15, **caractérisé en ce que** la mémoire électronique (M) est conçue sous la forme d'une carte à puce comprenant un corps de carte dans lequel sont incorporés la mémoire électronique (M) et une antenne, notamment une bobine de transpondeur.

18. Système selon la revendication 17 ainsi que la revendication 7 ou la revendication 13, **caractérisé en ce que** la carte à puce est fixée de manière amovible au vidéodisque (1) ou à la cassette (2).

19. Système selon la revendication 18, **caractérisé en ce que** la carte à puce est fixée au vidéodisque (1) ou à la cassette (2) au moyen d'une liaison enfichable.

20. Système selon la revendication 19, **caractérisé en ce que** la liaison enfichable présente un mécanisme, notamment un mécanisme à enclenchement rapide, pour verrouiller la carte à puce dans la liaison enfichable.

Fig. 1

M

IPI          IPS          IPC          IPP

| VN | VN | VN | VN |
| ... | ... | ... | ... |
| ... | ... | ... | ... |
| ... | ... | ... | ... |
| CS | CS | CS | CS |

CAL          IDP

| VN | VN |
| ... | ... |
| ... | ... |
| ... | ... |
| CS | CS |

Fig. 2

M

IPI

| VN |
| ... |
| ... |
| ... |
| CS |

CAL

| VN |
| ... |
| ... |
| ... |
| CS |

Fig. 5

IPI

IPS

IPC (IPC-Ref)

IPP (IPP-Ref)

IDP (IDP-Ref)

IMD

CAL

IDP (IDP-Ref)

IPP (IPP-Ref)

IPC (IPC-Ref)

IPS

IPI

(IPC + IPC-Ref)
(IPP + IPP-Ref)
(IDP + IDP-Ref)

IPS

IPC (IPC-Ref)

IPP (IPP-Ref)

IDP (IDP-Ref)

EP 1 544 672 B1

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5418355 A
- EP 1209517 A2
- EP 0727696 A1